(19) **Europäisches Patentamt / European Patent Office / Office européen des brevets**

(11) **EP 2 657 215 B1**

(12) **EUROPEAN PATENT SPECIFICATION**

(45) Date of publication and mention
of the grant of the patent:
**28.06.2017 Bulletin 2017/26**

(51) Int Cl.:
*C07C 29/151* (2006.01)     *C07C 31/04* (2006.01)
*C10K 1/34* (2006.01)     *C10K 1/32* (2006.01)
*C10K 1/00* (2006.01)     *C10K 3/06* (2006.01)

(21) Application number: **11864561.3**

(22) Date of filing: **28.04.2011**

(86) International application number:
**PCT/CN2011/073445**

(87) International publication number:
**WO 2012/145910 (01.11.2012 Gazette 2012/44)**

(54) **METHOD AND DEVICE FOR PRODUCING METHANOL**

VERFAHREN UND VORRICHTUNG ZUR METHANOLHERSTELLUNG

PROCÉDÉ ET DISPOSITIF DE PRODUCTION DE MÉTHANOL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**30.10.2013 Bulletin 2013/44**

(73) Proprietor: **Sichuan Daxing Energy Co., Ltd
Sichuan 610000 (CN)**

(72) Inventors:
 • **JIANG, Shanming
  Sichuan 610000 (CN)**
 • **WU, Yingzhong
  Sichuan 610000 (CN)**
 • **ZHANG, Guanghui
  Sichuan 610000 (CN)**
 • **LI, Yuanting
  Sichuan 610000 (CN)**
 • **ZHANG, Dongcao
  Sichuan 610000 (CN)**
 • **LI, Kebing
  Sichuan 610000 (CN)**

 • **ZHAO, Guangmei
  Sichuan 610000 (CN)**

(74) Representative: **Hirsch & Associés
137, rue de l'Université
75007 Paris (FR)**

(56) References cited:
**EP-A2- 0 200 880     WO-A1-2005/051872
CN-A- 1 373 113     CN-A- 1 373 113
CN-A- 102 079 689**

 • **LI KEBING ET AL: "New Technique of
Comprehensive Utilization of Coke Oven Gas and
Converter Coal Gas", HUAGONG JINZHAN -
CHEMICAL INDUSTRY AND ENGINEERING
PROGRESS, CHEMICAL INDUSTRY PRESS, CN,
vol. 29, no. S1, 1 January 2010 (2010-01-01), pages
325-327, XP008169502, ISSN: 1000-6613**
 • **LI KEBING ET AL.: 'New Technique of
Comprehensive Utilization of Coke Oven Gas and
Converter Coal Gas.' CHEMICAL INDUSTRY AND
ENGINEERING PROGRESS. 1000-6613 vol. 29,
no. S1, 2010, pages 325 - 327, XP008169502**

**Description**

**FIELD OF THE INVENTION**

The present invention relates to a field of chemical engineering, in particular relates to a method and device for producing methanol.

**BACKGROUND ART**

At present, a lot of raw materials, such as natural gas, coke oven gas, naphtha, heavy oil, coal or coke, are used for producing methanol. In most steel coke cogeneration enterprises, the coke oven gas produced by coking process is utilized to produce methanol. Since coke oven gas is a gas with high hydrogen content so that in the process of methanol production, the hydrogen to carbon ratio in the synthesis gas produced in the raw coke oven gas treatment far deviates from the theoretical value (the theoretical value of hydrogen to carbon ratio by volume F = (H$_2$ - CO$_2$) / (CO + CO$_2$) = 2.0 ∼ 2.05), resulting in that the hydrogen in the synthesis gas can not be fully utilized, and eventually be burned or exhausted. Since the useful elements can not be fully utilized, a waste of resources is caused, and at the same time, the efficiency of methanol production is lowered.

Document CN1373113 discloses a process for synthesizing methanol from coke oven gas without air separator and autothermal conversion characterized in that the coke oven gas is physically mixed with one or more gas sources with low hydrogen/carbon ratio.

Li Kebing et al., Chem Ind. Eng, Progress, 29, 325-327 (2010) disclose a technique of comprehensive utilization of coke oven gas and converter coal gas.

**SUMMARY OF THE INVENTION**

In view of the above, the present invention provides a method and device for producing methanol which, by making use of the converter gas generated in steelmaking process and the coke oven gas generated in coking process as raw materials, enables full use of the carbon element in the converter gas and the hydrogen element in the coke oven gas, saves resources, and improves economic efficiency.

To achieve the above object, the present invention provides the following technical solutions:

The present invention discloses a method for producing methanol, comprising:

purifying converter gas;

mixing the purified converter gas with coke oven gas to obtain mixed gas; and

reacting the mixed gas to produce methanol.

Preferably, the process of purifying converter gas comprises:

pressurizing the converter gas;

removing the mechanical water in the pressurized converter gas;

adsorbing the impurities from the mechanical water-removed converter gas; and

removing arsenic from the impurities-removed converter gas to obtain purified converter gas.

The process of reacting the mixed gas to produce methanol comprises:

desulfurizing the mixed gas;

reforming the desulfurized mixed gas using pure oxygen catalyzed partial oxidation reforming process to obtain synthesis gas; and

reacting the synthesis gas under the condition of 215 °C ∼ 280 °C and 5.5 Mpa ∼ 6.5 Mpa to produce methanol.

Preferably, the process of mixing the purified converter gas and the coke oven gas is mixing after adjusting the mixing ratio of the purified converter gas and the coke oven gas according to the hydrogen to carbon ratio in the synthesis gas, and the hydrogen to carbon ratio by volume in the synthesis gas is $(H_2 - CO_2) / (CO + CO_2 = 2.0 \sim 2.05$.

Preferably, before desulfurizing the mixed gas, further comprising pressurizing the mixed gas to a pressure of 2.2 Mpa $\sim$ 2.8 Mpa.

Preferably, making use of the reaction of the synthesis gas under the condition of 215 °C $\sim$ 280 °C and 5.5 Mpa $\sim$ 6.5 Mpa to produce methanol.

Preferably, reacting the synthesis gas to produce methanol and at the same time adding water to supplement the water lost in the reaction, and the ratio by weight between the amount of the makeup water and the yield of methanol is 0.90 $\sim$ 0.96 : 1.

Preferably, further comprising rectifying the methanol to obtain refined methanol.

The present invention also discloses an device for producing methanol, comprising:

a device for purifying converter gas connected to the converter gas exhaust port; a mixing device connected to the coke oven gas exhaust port and the exhaust port of the device for purifying converter gas;
a synthesis device connected to the exhaust port of the mixing device; and
a controller connected to the synthesis device, the coke oven gas exhaust port and the exhaust port of the device for purifying converter gas, wherein the controller adjusts the flow rate of the purified converter gas and the coke oven gas based on real-time monitoring of the hydrogen to carbon ratio in the synthesis gas so as to control the mixing ratio of the purified converter gas and the coke oven gas.

Preferably, the synthesis device comprises:

a desulfurizing device connected to the exhaust port of the mixing device;
a reforming device connected to the exhaust port of the desulfurizing device; and
a synthesis tower connected to the exhaust port of the reforming device.

As can be seen from the above technical solutions, according to the method and device disclosed in the embodiments of the present invention, the by-product gas - converter gas produced by steelmaking process is made to go through a purification process in order that it can be mixed with the coke oven gas produced by coking process at a suitable proportion so that the hydrogen to carbon ratio in the synthesis gas produced by the desulfurization and reformation of the mixed gas conforms to the requirement of theoretical value and so the production efficiency is improved. Since converter gas has a relatively high content of carbon element, while coke oven gas has a relatively high content of hydrogen element, the hydrogen to carbon ratio in the synthesis gas can be adjusted by adding a certain proportion of converter gas. The mixing ratio of converter gas and coke oven gas is adjusted real-timely according to the variation of the hydrogen to carbon ratio in the synthesis gas monitored real-timely, and the mixing ratio after adjustment is so that the hydrogen to carbon ratio in the synthesis gas conforms to the requirement of $F = (H_2 - CO_2) / (CO + CO_2) = 2.0 \sim 2.05$.

Since converter gas and coke oven gas are both by-product gas in industrial production processes, the addition of converter gas can, on one hand, increase the use ratio of useful elements in the raw material, and on the other hand, the utilization of industrial by-product gas as raw material can effectively reduce the emission of waste gas and reduce the pollution from waste gas to the environment. In addition, the utilization of the converter gas discharged as waste gas in steelmaking process as raw material can decrease production cost and increase economic benefit.

## BRIEF DESCRIPTION OF THE DRAWINGS

To more clearly illustrate the embodiments of the present invention or the technical solutions in the prior art, the following briefly describes the accompanying drawings to be used in describing the embodiments or the prior art. Obviously, the accompanying drawings are merely some embodiments of the present invention. For those of ordinary skill in the art, other accompanying drawings may be obtained without creative efforts according to these accompanying drawings.

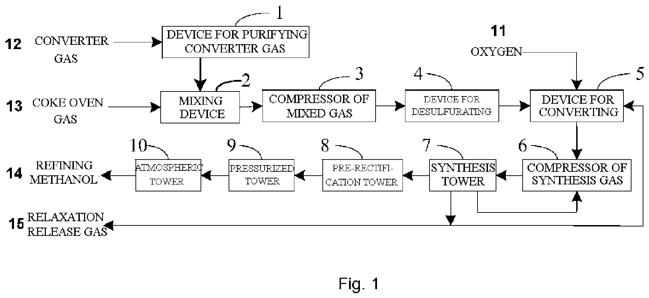

Fig. 1 is a flow diagram for a methanol production process disclosed in an embodiment of the present invention.

## DETAILED DESCRIPTION

The embodiment of the present invention discloses a method and device for producing methanol, in which the by-product gas - converter gas produced by steelmaking process is made to go through purification and is mixed with the coke oven gas produced by coking process at a suitable proportion so that the hydrogen to carbon ratio in the synthesis gas produced by the desulfurization and reformation of the mixed gas conforms to the requirement of theoretical value, and then when the synthesis gas is used to produce methanol, the use ratio of useful elements in the raw materials is increased, energy is saved and pollution is reduced at the same time.

For a further understanding of the present invention, the preferred embodiments of the present invention will be described hereinafter clearly and completely with reference to the accompanying drawings and examples, however, it should be appreciated that these descriptions are merely for further illustrating the features and advantages of the present invention.

The present invention discloses a method for producing methanol, comprising:

purifying converter gas;
mixing the purified converter gas with coke oven gas to obtain mixed gas; and
reacting the mixed gas to produce methanol.

Converter gas is a mixed gas containing carbon monoxide and a small amount of carbon dioxide produced by the carbon in the molten iron and the oxygen blown in under high temperature during converter steelmaking process. The recycled top-blown oxygen converter gas contains 60% ~ 80% of carbon monoxide and 15% ~ 20% of carbon dioxide as well as nitrogen, hydrogen, trace amount of oxygen, sulfur, phosphorus, arsenic, fluorine and other impurities. The generated amount of converter gas in a smelting process is not balanced and the ingredients also vary. Usually, converter gas is only used as fuel after purification. The purification process adopts electric-process dust removal or wet-process dust removal in an effort only to remove the dusts in converter gas to a level of less than 20 mg/Nm$^3$, which belongs to rough purification, and the sulfur, phosphorus, arsenic, fluorine and other minor amount of impurities cannot be removed. The sulfur, phosphorus, arsenic, fluorine and other impurities are harmful to the catalyst during methanol production process, so only after these impurities are removed can the converter gas be used as a raw material for producing methanol.

The converter gas has a temperature of as high as 1450 °C ~ 1500 °C when ejected out from furnace mouth and carries a lot of iron oxide dust. It can only be used after cooling down and dust removal, and there are two types of purification, i.e. wet process and dry process. The embodiments of the present invention adopt wet-process dust removal and cooling method, that is, dust removal with water, in which rough purification is firstly conducted to the converter gas, and then the temperature swing adsorption purification technology is adopted to remove the sulfur, phosphorus, arsenic, fluorine and other impurities. Specifically, the process of purification to remove impurities includes:

allowing the converter gas to enter a blower at a flow rate of about 3000 Nm$^3$/h under a pressure of about 10 kpa, and pressurizing the converter gas to about 45 to 55 kpa, preferably 50 kpa at the blower;

having the pressurized converter gas to go through a gas liquid separator so as to remove the mechanical water in it, these mechanical water coming from the residual moisture in the converter gas after dust removal with water;

adsorbing the impurities in the mechanical water-removed converter gas; and

conducting arsenic removal by having the impurities adsorbed converter gas to go through a refining unit made up of an arsenic remover to obtain purified converter gas, preferably, the arsenic-removal agent in this process is for a single use.

Preferably, the process of adsorbing the impurities in the converter gas in the embodiments of the present invention is conducted in an adsorption purification unit made up of three adsorbers, and the adsorbing agent in the adsorbers can selectively adsorb the PH$_3$, AsH$_3$, HF and other impurities in the converter gas. Specifically, the converter gas goes through a single adsorber at a same time, and then when the impurities adsorbed by the adsorbing agent in an adsorber are approaching saturation, the converter gas is passed into another adsorber in which the adsorbing agent has been regenerated to conduct impurities adsorption, and so on. In adsorption purification unit, since the impurity components adsorbed on the adsorbing agent will be liberated upon heating, each adsorber will orderly go through the steps of adsorbing impurities by adsorbing agent, heating flushing, cold blowing and the like at different time. Preferably, in this embodiment, the superheated steam at about 350 °C and 0.2 Mpa produced by heating with an electric heater is used to hot blow the adsorbing agent, then the generated waste gas is cooled down and separated into waste water and waste gas, the waste water is discharged through sewage pipe network, and the waste gas is released into atmosphere by flaring; after flushing the adsorbing agent, the recycled nitrogen gas is used to carry out cold blow to the adsorbing agent so that it is dry cooled to normal temperature, which process is the regeneration process of adsorbing agent, and the regenerated adsorbing agent can be further recycled to adsorb the impurities in converter gas.

The purified converter gas is mixed with coke oven gas at a suitable proportion to obtain mixed gas. The role of the addition of converter gas into coke oven gas is to adjust the hydrogen to carbon ratio in the synthesis gas so that an optimal ratio for producing methanol is reached. A too low hydrogen to carbon ratio, i.e., the addition of too much converter gas, may cause problems such as coke deposition, aging and deactivation of the catalyst at the time of methanol synthesis; a too low hydrogen to carbon ratio, i.e., the addition of too little converter gas, can reduce the methane content in the synthesis gas and prevent coke deposition of the catalyst from happening, but may increase the resistance for the generation of methanol from the synthesis gas, increase energy consumption, reduce the production capacity of the device, and lead to a low synthetic ratio of methanol. Consequently, the mixing ratio of converter gas and coke oven gas must be strictly controlled.

The mixing ratio of converter gas and coke oven gas in this embodiment is adjusted real-timely according to the real-time monitoring of the hydrogen to carbon ratio in the synthesis gas. The theoretical value of hydrogen to carbon ratio by volume in the synthesis gas is $F = (H_2 - CO_2) / (CO + CO_2) = 2.0 \sim 2.05$, as a result, preferably, the purified converter gas and the coke oven gas are mixed at a proportion by volume of 0.8~1.2 : 9.2-8.8 between the purified converter gas and the coke oven gas, and more preferably, the purified converter gas and the coke oven gas are mixed at a proportion by volume of 0.95~1.05 : 9.05~8.95 between the purified converter gas and the coke oven gas.

It should be noted that since the ingredients in the converter gas and the coke oven gas are changing with time, accordingly, the mixing ratio of the purified converter gas and the coke oven gas needs to be adjusted real-timely. The manner for mixing ratio adjustment is real-timely monitoring the ingredients in the purified converter gas and the coke oven gas and then real-timely adjusting the mixing ratio of the purified converter gas and the coke oven gas in combination with the monitoring result of the hydrogen to carbon ratio in the synthesis gas; it may alternatively be utilizing the empirical value for the ingredients in the purified converter gas and the coke oven gas obtained in the production process and combining the monitoring result of the hydrogen to carbon ratio in the synthesis gas to real-timely adjust the mixing ratio of the purified converter gas and the coke oven gas. In the present invention, the former is used, however, the selection of the manner for mixing ratio adjustment does not affect the scope of protection of the present invention. In this embodiment, the process of making use of the reaction of the mixed gas to produce methanol includes:

pressurizing the mixed gas to a pressure of 2.2 Mpa ~ 2.8 Mpa, preferably 2.5 Mpa, the pressurization to the mixed gas may be done by centrifugal turbine compressor or reciprocating compressor or other technologies generally known by those skilled in the art;

desulfurizing the pressurized mixed gas to lower the total sulfur in the mixed gas to be less than 0.1 ppm, and there are many specific ways to conduct the desulfurization: for example, activated carbon can be used to remove the tar and other substances in the coke oven gas, since the forms of sulfur in coke oven gas are complicated, two times of hydroconversion is necessary so that the various forms of organic sulfur are converted into inorganic sulfur, then zinc oxide at moderate temperature and zinc oxide at normal temperature are used to remove inorganic sulfur so that the total sulfur is less than 0.1 ppm; alternatively, a method of physical absorption by solvent can be used to remove the majority of sulfides in the mixed gas, then hydrolysis and dry-process fine desulfurizer such as ferric oxide, zinc oxide or activated carbon are adopted to remove the possible residual trace amount of sulfides, so that the requirement for methanol synthesis is met. When the method of physical absorption by solvent is adopted, the solvent can be selected from the group consisting of the combined solution of methanol, sulfolane and diisopropanolamine, the polyethylene glycol dimethyl ether or the propylene carbonate. The first way is chosen in this embodiment, however, for the way of desulfurization, other methods and other substances generally known by those skilled in the art can also be adopted;

reforming the desulfurized mixed gas using pure oxygen catalyzed partial oxidation reforming process to obtain synthesis gas, the synthesis gas is reformed from the methane and a small amount of multi-carbon hydrocarbon in the mixed gas, it contains carbon monoxide, carbon dioxide and hydrogen gas, and the hydrogen to carbon ratio by volume in the synthesis gas is $F = (H_2 - CO_2) / (CO + CO_2) = 2.0 \sim 2.05$, preferably, the hydrogen to carbon ratio by volume in the synthesis gas is $F = (H_2 - CO_2) / (CO + CO_2) = 2.01 \sim 2.03$, and more preferably, the hydrogen to carbon ratio by volume in the synthesis gas is $F = (H_2 - CO_2) / (CO + CO_2) = 2.02$; and

afterwards, making use of the reaction of the synthesis gas under the condition of 215 °C ~ 280 °C and 5.5 Mpa ~ 6.5 Mpa to produce methanol, the reaction temperature of the synthesis gas is preferably 230 °C ~ 260 °C, more preferably, 240 °C ~ 250 °C, and the reaction pressure of the synthesis gas is preferably 5.8 Mpa ~ 6.2 Mpa, more preferably, 6.0 Mpa.

The methanol synthesis reaction is a strongly exothermic reaction, with the addition of converter gas, the concentration of CO and $CO_2$ will increase, the temperature in the synthesis tower will rise rapidly and exceed the optimal operating temperature range of the catalyst for the synthesis. In case of long-term high temperature operation, the grain growth of the catalyst will be accelerated, the surface area will be decreased, accordingly, the aging of the catalyst will be expedited, so the bed temperature must be kept lower than 280 °C, on the other hand, due to that when the bed temperature is lower than 215 °C, the catalyst might suffer from wax deposit reaction, therefore, the optimal temperature range is 215 °C ~ 280 °C.

From calculation, it is found that, due to the increase of the concentration of CO and $CO_2$ brought about by the addition of converter gas, the methanol production will increase for about 3.28 t/h. In this embodiment, the methanol synthesis tower is shell-and-tube synthesis tower. According to measurement and calculation, one ton of methanol increment will yield about 0.93 tons of steam, on this account, in this embodiment, in order to control the temperature rise in the synthesis tower, at the same time of making use of the reaction of the synthesis gas to produce methanol, water is used to make up the loss of water in the reaction, i.e., the water taken away by the by-product steam of the shell boiler water in the synthesis tower. The ratio by weight between the amount of the boiler makeup water and the yield of methanol is preferably 0.90 ~ 0.96 : 1, more preferably 0.93 : 1, that is, the amount of makeup water is about 3.15 t/h.

The methanol produced from the reaction of the synthesis gas is crude methanol, as a result, in this embodiment, three-tower (made up of pre-rectification tower, pressurized tower and atmospheric tower) rectification technology is further adopted to conduct rectification to the methanol so as to obtain refined methanol, afterwards, the methanol is sent to finished product tank farm for storage.

The method for producing methanol disclosed in the embodiments of the present invention uses the by-product gas - converter gas in the steelmaking production process that has high CO content in its composition and is otherwise burned as waste gas to be a raw material for the methanol production. Since CO is also a valuable chemical raw material, its burning as waste gas not only leads to environmental pollution but also represents a waste of resource. Consequently, based on the fact that coke oven gas has high content of hydrogen element and low content of carbon element while converter gas has high content of carbon element and low content of hydrogen element, mixing the two gases at a suitable proportion and using as the raw material for methanol production not only resolves the problems such as incomplete hydrogen consumption, low methanol synthetic ratio and resource wastage as caused by the single use of coke oven gas as raw material, but also reduces the pollution to the environment, in addition, from an economic standpoint, using waste gas as industrial raw material also reduces production cost.

The present invention also provides an device for producing methanol (see Fig. 1), comprising:

a device for purifying converter gas 1 connected to the converter gas exhaust port;

a mixing device 2 connected to the coke oven gas exhaust port and the exhaust port of the device for purifying converter gas 1, an opening is arranged at the coke oven gas conduit to introduce in the purified converter gas, the coke oven gas mixes with the purified converter gas to produce mixed gas;

a mixed gas compressor 3 connected to the mixing device 2, the mixed gas compressor 3 pressurizes the mixed gas to a pressure of 2 Mpa ~ 2.8 Mpa;

a desulfurizing device 4 connected to the exhaust port of the mixed gas compressor 3;

a reforming device 5 connected to the exhaust port of the desulfurizing device 4, in the reforming device 5, the desulfurized mixed gas is reformed into a synthesis gas containing carbon monoxide and hydrogen, and the hydrogen to carbon ratio by volume in the synthesis gas is $(H_2 - CO_2) / (CO + CO_2) = 2.0 \sim 2.05$;

a synthesis gas compressor 6 connected to the exhaust port of the reforming device 5, the synthesis gas compressor 6 pressurizes the synthesis gas to a pressure of 5.5 Mpa ~ 6.5 Mpa;

a shell-and-tube synthesis tower 7 connected to the exhaust port of the synthesis gas compressor 6, the synthesis tower 7 uses the reaction of the synthesis gas to produce methanol; and

a controller connected to the reforming device 5, the coke oven gas exhaust port and the exhaust port of the device for purifying converter gas, the controller adjusts the flow rate of the purified converter gas and the coke oven gas based on the real-time monitoring of the hydrogen to carbon ratio in the synthesis gas so as to control the mixing ratio of the purified converter gas and the coke oven gas, the mixing ratio between the purified converter gas and the coke oven gas is 0.8~1.2 : 9.2~8.8 by volume.

Of which, the desulfurizing device 4, the reforming device 5, the synthesis gas compressor 6 and the synthesis tower 7 in this embodiment are collectively referred to as the synthesis device for producing methanol by making use of the reaction of mixed gas.

In addition, further comprising a pre-rectification tower 8, a pressurized tower 9 and an atmospheric tower 10 connected to the synthesis tower 7 and used to conduct rectification to the methanol produced by the synthesis tower 7.

Those skilled in the art will understand that the mixing of the purified converter gas and the coke oven gas can occur at the coke oven gas conduit and can also occur in the mixed gas compressor or other mixing device can be arranged, however, the selection of the mixing device does not affect the scope of protection of the present invention.

Hereinafter, the device and process flow for methanol production disclosed in this embodiment will be described in reference to Fig. 1 and method embodiments:

The converter gas from steelmaking process is allowed to go through a wet-process dust removal device, a blower, a gas liquid separator, an adsorption purification unit and a refining unit in the device for purifying converter gas 1, then the converter gas is allowed to cool down, and the dust as well as the impurities such as sulfur, phosphorus, arsenic, fluorine and the like in the converter gas that may do harm to the catalysts for synthesis and reforming are removed so that a purified converter gas satisfying the reaction requirement is obtained. The criteria for the purified converter gas is as follows: $PH_3$ < 1 ppm; COS < 1 ppm; HF < 0.1 ppm; $AsH_3$ < 5 ppb; $H_2S$ < 1 ppm, the converter gas from the converter is treated to meet the above criteria and then mixed with the coke oven gas from the coke oven to obtain the mixed gas.

Before mixing with the purified converter gas, the coke oven gas is pretreated in cokeoven plant. The said pretreatment process is:

removing the tar in the coke oven gas to be less than 50 mg/$Nm^3$ by an electrical tar precipitator set downstream the blower;

removing the ammonia in the coke oven gas to be less than 50 mg/$Nm^3$ by acid cleaning process in an ammonium sulfate section downstream the blower;

removing the naphthalene in the coke oven gas to be less than 100 mg/$Nm^3$ by cyclically spraying condensate using a cross-tube indirect cooler;

reducing the benzenes in the coke oven gas to be less than 2 g/$Nm^3$ by cyclically washing with tar wash oil; and

reducing the hydrogen sulfide and hydrogen cyanide in the coke oven gas to be less than 20 mg/$Nm^3$ by PDS method.

The coke oven gas that has gone through the aforementioned pretreatment and the purified converter gas are mixed in proportion at mixed gas compressor and compressed to a pressure of 2.2 Mpa ~ 2.8 Mpa, then they are sent into desulfurizing device 4 for the fine desulfurization of the mixed gas to remove total sulfur to be 0.1 ppm or less. The desulfurized gas is mixed with oxygen from outside and is allowed to experience reformation in reforming device 5 using pure oxygen catalyzed partial oxidation process to reform the methane and the small amount of multi-carbon hydrocarbon in the mixed gas into useful ingredients for methanol synthesis: carbon monoxide, carbon dioxide and hydrogen gas, i.e., the synthesis gas necessary for methanol production. Then the synthesis gas is pressurized to a pressure of 5.5 Mpa ~ 6.5 Mpa by synthesis gas compressor 6. The pressurized synthesis gas is allowed to react in synthesis tower 7 to produce methanol, and the reaction temperature is controlled to the range of 215 °C ~ 280 °C by making up a certain amount of boiler water into the synthesis tower, and then the crude methanol is rectified by three-tower rectification technology to obtain refined methanol.

Of which, the means for the control of the mixing ratio of purified converter gas and coke oven gas is real-timely monitoring the hydrogen to carbon ratio in the synthesis gas and then real-timely adjusting the flow rate of the purified converter gas and the coke oven gas according to the variation of the hydrogen to carbon ratio. During the production process, the gas not completely reacted in the synthesis tower 7 has several places to go, for example, going back into the synthesis gas compressor 6 as recycle gas and compressed again for a second time reaction, going back into the reforming device 5 as a heat source for preheating furnace or heating furnace, acting as a heat source for heating coke oven, or extracting the hydrogen in purge gas to use as hydrogen source, that is, allowing the purge gas to go through pressure swing adsorption device to extract hydrogen, the concrete measure for which is generally known by those skilled in the art, so detailed description thereof is omitted here, however, the processing mode to the gas not completely reacted in the synthesis tower does not affect the scope of protection of the present invention.

It should be noted that, the main reactions for the desulfurization conducted in desulfurizing device 4 include:

$$RSH + H_2 = H_2S + RH;$$

$$RSR + 2H_2 = H_2S + 2RH;$$

$$R_1\text{-}S\text{-}R_2 + 2H_2 = R_1H + R_2H + H_2S;$$

$$COS + H_2 = H_2S + CO;$$

$$CS_2 + 2H_2O = 2H_2S + CO + CO_2;$$

$$H_2S + FeO = FeS + H_2O;$$

$$H_2S + ZnO = ZnS + H_2O.$$

Where, thioalcohol RSH, thioether RSR, $R_1\text{-}S\text{-}R_2$ and $CS_2$ are all impurities in coke oven gas.

The main reactions for the pure oxygen catalyzed partial oxidation process conducted in reforming device 5 include:

$$2H_2 + O_2 = H_2O + 115.48 \text{ kcal};$$

$$2CH_4 + O_2 = 2CO, + 4H_2 + 17.0 \text{ kcal};$$

$$CH_4 + H_2O = CO + 3H_2 - 49.3 \text{ kcal};$$

$$CH_4 + CO_2 = 2CO, + 2H_2 - 59.1 \text{ kcal};$$

$$CO + H_2O = CO_2 + H_2 + 9.8 \text{ kcal}.$$

Where, the CO, $CO_2$ and $H_2$ produced after the reaction are just the main ingredients of the synthesis gas, and the hydrogen to carbon ratio in the synthesis gas should be controlled to $2.0 \sim 2.05$.

The main reactions for the methanol production process by making use of the reaction of the synthesis gas conducted in synthesis tower 7 include:

$$CO + 2H_2 = CH_3OH + Q;$$

$$CO_2 + 3H_2 = CH_3OH + H_2O + Q.$$

For a better understanding of the present invention, the method for producing methanol provided in the present invention will be described here below in conjunction with specific examples.

Example 1

Taking the coke oven gas and converter gas produced by steel coke cogeneration enterprises as raw material, at a certain moment, the composition of the raw material is as follows:

The content of the various components in coke oven gas:

| Item | $H_2$ | CO | $CO_2$ | $CH_4$ | $N_2$ | $C_mH_n$ | $O_2$ | $\Sigma$ |
|------|-------|------|--------|--------|-------|----------|-------|----------|
| V% | 56.52 | 7.54 | 2.96 | 26.91 | 2.80 | 2.75 | 0.52 | 100 |

The content of the various components in converter gas:

| Item | $H_2$ | CO | $CO_2$ | $N_2$ | $O_2$ | $PH_3$ | COS | HF | $AsH_3$ | $H_2S$ |
|------|-------|------|--------|-------|-------|--------|------|-------|---------|--------|
| V% | 1.5 | 59 | 18. | 20. | 0.4 | 600ppm | 7ppm | 1ppm | 0.1ppm | 30ppm |

The ingredients and impurities in converter gas before and after purification:

| Item | $H_2$ | CO | $CO_2$ | $N_2$ | $O_2$ | $PH_3$ | COS | HF | $AsH_3$ | $H_2S$ |
|------|-------|------|--------|-------|-------|--------|------|-------|---------|--------|
| Before | 1.5 | 59 | 18. | 20. | 0.4 | 600ppm | 7pp | 1ppm | 0.1pp | 30ppm |

(continued)

| Item | H_2 | CO | CO_2 | N_2 | O_2 | PH_3 | COS | HF | AsH_3 | H_2S |
|---|---|---|---|---|---|---|---|---|---|---|
| After | 3.5 | 63. | 13. | 19. | 0.2 | 1ppm | 1pp | 0.1 ppm | 5ppb | 1ppm |

The composition of coke oven gas and the reformed synthesis gas before the addition of converter gas:

| Item | $H_2$ | CO | $CO_2$ | $CH_4$ | $N_2$ | $C_mH_n$ | $O_2$ | $\Sigma$ |
|---|---|---|---|---|---|---|---|---|
| Coke oven gas | 56.52 | 7.54 | 2.96 | 26.91 | 2.80 | 2.75 | 0.52 | 100 |
| Synthesis gas | 71.42 | 11.42 | 11.86 | 0.83 | 4.13 | 0.00 | 0.34 | 100 |

At this moment, according to the on-line analysis of the synthesis gas necessary for methanol production reformed from the single use of coke oven gas, the effective ingredients have the following proportions in the synthesis gas: $H_2$, 71.42%; CO, 11.42%; $CO_2$, 11.86%. The temperature of the synthesis gas before entering the synthesis gas compressor is controlled below 40 °C and the pressure is controlled at about 2.0 Mpa, at which time, the hydrogen to carbon ratio of the synthesis gas is F = $(H_2 - CO_2) / (CO + CO_2)$ = (71.42 - 11.86) / (11.42 + 11.86) = 2.56. It can be seen that at this moment, the hydrogen content in the synthesis gas is on the high side and adding carbon is necessary. The carbon source needed to add is converter gas, and the useful ingredients in the converter after purification include: CO, 63.22%; $CO_2$, 13.30%; $H_2$, 3.50%. The temperature of the purified converter gas is controlled below 40 °C, and the pressure is controlled below 0.01 Mpa. Assume that the flow rate of the synthesis gas at certain moment is 30000 $Nm^3$/h, according to the requirement of rational hydrogen to carbon ratio, F takes a value of 2.02, if the added converter gas is X $Nm^3$/h, then the computation expression is as follows:

$$F = (H2 - CO2) / (CO + CO2) = \{(30000*71.42 + 3.5*X) - (30000*11.86 + 13.30*X)\} / \{30000*(11.42 + 11.86) + (63.22 + 13.30)*X\} = 2.02;$$

$$X = 2288 \ Nm^3/h;$$

So, the flow rate of the converter gas needed at the moment is calculated to be 2288 $Nm^3$/h.

The comparison of the ingredients and hydrogen to carbon ratio in the synthesis gas between before and after the addition of converter gas:

| Item | $H_2$ | CO | $CO_2$ | $CH_4$ | $N_2$ | $O_2$ | Hydrogen to carbon ratio |
|---|---|---|---|---|---|---|---|
| Before, V% | 71.42 | 11.42 | 11.86 | 0.83 | 4.13 | 0.34 | 2.56 |
| After, V% | 66.61 | 15.09 | 11.96 | 0.79 | 5.17 | 0.38 | 2.02 |

The optimal introduction amount of the converter gas is adjusted with time according to the method described above, the synthesis gas produced from the reformation of the mixed gas is pressurized to a pressure of 6.0 Mpa by synthesis gas compressor and further sent to methanol synthesis tower to obtain crude methanol. The synthesis of methanol adopts a 6.0 Mpa low-pressure synthesis technology, the synthesis tower used is of shell and tube type, the synthesized crude methanol is rectified by three-tower (made up of pre-rectification tower, pressurized tower and atmospheric tower) rectification technology to prepare refined methanol, and the product refined methanol is sent to finished product tank farm for storage. Tests indicate that the quality of the methanol product produced from this process achieves the level of high-class product according to GB338-2004.

The comparison of the content of methanol purge gas between before and after the addition of converter gas:

| Item | $H_2$ | CO | $CO_2$ | $O_2$ | $CH_4$ | $N_2$ |
|---|---|---|---|---|---|---|
| Before, V% | 77.59 | 2.96 | 7.65 | 0.13 | 4.16 | 7.51 |
| After, V% | 63.70 | 5.79 | 10.80 | 0.26 | 4.87 | 14.58 |

It can be seen from the data above that after the addition of converter gas, the content of $H_2$ in the purge gas in respect to the total amount of purge gas decreases 13.8% from 77.59% to 63.70%, indicating that with the addition of converter gas, the use ratio of $H_2$ increases and the discharge amount decreases. In addition, in this example, due to that the use ratio of raw material is increased and waste gas is utilized as raw gas, the economic benefit is improved and at the same time the pollution to environment is reduced.

**Claims**

1. Method for producing methanol, **characterized in that** it comprises:

   • purifying converter gas;
   • mixing the purified converter gas with a coke oven gas to obtain mixed gas; and
   • reacting the mixed gas to produce methanol,

   wherein the process of reacting the mixed gas to produce methanol comprises:

   • desulfurizing the mixed gas;
   • reforming the desulfurized mixed gas using pure oxygen-catalyzed partial oxidation reforming process to obtain synthesis gas;
   • reacting the synthesis gas under the condition of 215°C~280°C and 5.5 MPa ~ 6.5 MPa to produce methanol, and

   wherein ingredients in the purified converter gas and the coke oven gas are real-timely monitored and then the mixing ratio of the purified converter gas and the coke oven gas is adjusted real-timely in combination with the monitoring result of the hydrogen to carbon ratio in the synthesis gas.

2. Method according to claim 1, **characterized in that**, the process of purifying the converter gas comprises:

   • pressurizing the converter gas;
   • removing mechanical water from the pressurized converter gas;
   • adsorbing impurities from the mechanical water-removed converter gas; and

   removing arsenic from the impurities-removed converter gas to obtain purified converter gas.

3. Method according to claim 1, **characterized in that**, the process of mixing the purified converter gas and the coke oven gas is performed by mixing after adjusting the mixing ratio of the purified converter gas and the coke oven gas according to the hydrogen to carbon ratio in the synthesis gas, the hydrogen to carbon ratio by volume in the synthesis gas being ($H_2$ - $CO_2$) / (CO + $CO_2$) = 2.0 ~ 2.05.

4. Method according to claim 1, **characterized in that**, before desulfurizing the mixed gas, the method further comprises pressurizing the mixed gas to a pressure of 2.2 MPa ~ 2.8 MPa.

5. Method according to claim 1, **characterized in that**, reacting the synthesis gas under the condition of 215°C~280°C and 5.5 MPa ~ 6.5 MPa to produce methanol.

6. Method according to claim 5, **characterized in that**, reacting the synthesis gas to produce methanol and at the same time adding water to supplement the moisture lost in the reaction, and the ratio by weight between the amount of the added water and the yield of methanol is 0.90 ~ 0.96 : 1.

7. Method according to any of the claims 1 to 6, **characterized in** further comprising rectifying the methanol to obtain refined methanol.

8. Device for producing methanol, **characterized in that**, comprising:

   • a device for purifying converter gas connected to the converter gas exhaust port; a mixing device connected to the coke oven gas conduit and the exhaust port of the device for purifying converter gas;
   • a synthesis device connected to the exhaust port of the mixing device; and
   • a controller connected to the synthesis device, the coke oven gas conduit and the exhaust port of the device for purifying converter gas,

wherein the controller adjusts the flow rate of the purified converter gas and the coke oven gas based on the real-time monitoring of the hydrogen to carbon ratio in the synthesis gas so as to control the mixing ratio of the purified converter gas and the coke oven gas.

9. Device according to claim 8, **characterized in that**, the synthesis device comprises:

• a desulfurizing device connected to the exhaust port of the mixing device;
• a reforming device connected to the exhaust port of the desulfurizing device; and
• a synthesis tower connected to the exhaust port of the reforming device.

**Patentansprüche**

1. Verfahren zur Methanolherstellung, **dadurch gekennzeichnet, dass** es Folgendes umfasst:

• Reinigen eines Konvertergases;
• Mischen des gereinigten Konvertergases mit einem Koksofengas, um Mischgas zu erhalten; und
• Umsetzung des Mischgases, um Methanol herzustellen,

wobei das Verfahren zur Umsetzung des Mischgases, um Methanol herzustellen, Folgendes umfasst:

• Entschwefeln des Mischgases;
• Reformieren des entschwefelten Mischgases durch partieller Oxidation durch reinen Sauerstoff als Reformierungsverfahren, um ein Synthesegas zu erhalten;
• Umsetzung des Synthesegases unter der Bedingung von 215°C bis ~ 280°C und 5,5 bis ~ 6,5 MPa, um Methanol herzustellen, und

wobei Inhaltsstoffe in dem gereinigten Konvertergas und dem Koksofengas in Echtzeit überwacht werden, und dann das Mischungsverhältnis des gereinigten Konvertergases und des Koksofengases in Echtzeit in Kombination mit dem Überwachungsergebnis des Wasserstoff-zu-Kohlenstoff-Verhältnisses in dem Synthesegas angepasst wird.

2. Verfahren nach Anspruch 1, **dadurch gekennzeichnet, dass** das Verfahren des Reinigens des Konvertergases Folgendes umfasst:

• Unterdrucksetzen des Konvertergases;
• Entfernen von mechanischem Wasser aus dem unter Druck gesetzten Konvertergas;
• Absorbieren von Verunreinigungen aus dem Konvertergas, aus dem mechanisches Wasser entfernt wurde; und
• Entfernen von Arsen aus dem Konvertergas, aus dem Verunreinigungen entfernt wurden, um gereinigtes Konvertergas zu erhalten.

3. Verfahren nach Anspruch 1, **dadurch gekennzeichnet, dass** das Verfahren des Mischens des gereinigten Konvertergases und des Koksofengases durch Mischen nach dem Anpassen des Mischungsverhältnisses des gereinigten Konvertergases und des Koksofengases gemäß dem Wasserstoff-zu-Kohlenstoff-Verhältnis in dem Synthesegas durchgeführt wird, wobei das Wasserstoff-zu-Kohlenstoff-Verhältnis nach Volumen in dem Synthesegas $(H_2 - CO_2) / (CO + CO_2) = 2,0$ bis ~ 2,05 beträgt.

4. Verfahren nach Anspruch 1, **dadurch gekennzeichnet, dass** das Verfahren vor dem Entschwefeln des Mischgases ferner das Unterdrucksetzen des Mischgases auf einen Druck von 2,2 MPa bis ~ 2,8 MPa umfasst.

5. Verfahren nach Anspruch 1, **dadurch gekennzeichnet, dass** das Synthesegas unter der Bedingung von 215°C bis ~ 280°C und 5,5 bis ~ 6,5 MPa umgesetzt wird, um Methanol herzustellen.

6. Verfahren nach Anspruch 5, **dadurch gekennzeichnet, dass** das die Umsetzung von Synthesegas umgesetzt wird, um Methanol herzustellen, und gleichzeitig Wasser zugegeben wird, um die bei der Reaktion verloren gegangene Feuchtigkeit zu ergänzen, und das Verhältnis zwischen der Menge des zugegebenen Wassers und dem Methanolertrag nach Gewicht 0,90 ~ 0,96:1 beträgt.

**7.** Verfahren nach irgendeinem der Ansprüche 1 bis 6, **dadurch gekennzeichnet, dass** es ferner das Rektifizieren des Methanols umfasst, um veredeltes Methanol zu erhalten.

**8.** Vorrichtung zur Methanolherstellung, **dadurch gekennzeichnet, dass** es Folgendes umfasst:

• eine Vorrichtung zum Reinigen von Konvertergas, die mit der Konvertergas-Auslassöffnung verbunden ist; eine Mischvorrichtung, die mit der Koksofengasleitung und der Auslassöffnung der Vorrichtung zum Reinigen von Konvertergas verbunden ist;
• eine Synthesevorrichtung, die mit der Auslassöffnung der Mischvorrichtung verbunden ist; und
• eine Steuerung, die mit der Synthesevorrichtung, der Koksofengasleitung und der Auslassöffnung der Vorrichtung zum Reinigen von Konvertergas verbunden ist,

wobei die Steuerung die Strömungsgeschwindigkeit des gereinigten Konvertergases und des Koksofengases, auf der Grundlage der Echtzeitüberwachung des Wasserstoff-zu-Kohlenstoff-Verhältnisses in dem Synthesegas anpasst, um das Mischungsverhältnis des gereinigten Konvertergases und des Koksofengases zu steuern.

**9.** Vorrichtung nach Anspruch 8, **dadurch gekennzeichnet, dass** die Synthesevorrichtung Folgendes umfasst:

• eine Entschwefelungsvorrichtung, die mit der Auslassöffnung der Mischvorrichtung verbunden ist;
• eine Reformierungsvorrichtung, die mit der Auslassöffnung der Entschwefelungsvorrichtung verbunden ist; und
• einen Syntheseturm, der mit der Auslassöffnung der Reformierungsvorrichtung verbunden ist.

**Revendications**

**1.** Procédé de production de méthanol, **caractérisé en ce qu'**il comprend :

• la purification d'un gaz de convertisseur ;
• le mélange du gaz de convertisseur purifié avec un gaz de cokerie pour obtenir un mélange de gaz ; et
• la mise en réaction du mélange de gaz pour produire du méthanol,

dans lequel le procédé de mise en réaction du mélange de gaz pour produire du méthanol comprend :

• la désulfuration du mélange de gaz ;
• le reformage du mélange de gaz désulfurisé en utilisant un procédé de reformation par oxydation partielle catalysée par de l'oxygène pur pour obtenir un gaz de synthèse ;
• la mise en réaction du gaz de synthèse dans les conditions de 215 °C à 280 °C et de 5,5 MPa à 6,5 MPa pour produire du méthanol, et

dans lequel les ingrédients dans le gaz de convertisseur purifié et le gaz de cokerie sont surveillés en temps réel et ensuite le rapport de mélange du gaz de convertisseur purifié et du gaz de cokerie est ajusté en temps réel en association avec le résultat de surveillance du rapport d'atomes d'hydrogène aux atomes de carbone dans le gaz de synthèse.

**2.** Procédé selon la revendication 1, **caractérisé en ce que** le procédé de purification du gaz de convertisseur comprend :

• la pressurisation du gaz de convertisseur ;
• l'élimination de l'eau mécanique du gaz de convertisseur pressurisé ;
• l'absorption des impuretés du gaz de convertisseur dont l'eau mécanique a été éliminée ; et
• l'élimination de l'arsenic du gaz de convertisseur dont les impuretés ont été éliminées pour obtenir un gaz de convertisseur purifié.

**3.** Procédé selon la revendication 1, **caractérisé en ce que** le procédé de mélange du gaz de convertisseur purifié et du gaz de cokerie est réalisé par mélange après ajustement du rapport de mélange du gaz de convertisseur purifié et du gaz de cokerie selon le rapport d'atomes d'hydrogène aux atomes de carbone dans le gaz de synthèse, le rapport d'atomes d'hydrogène aux atomes de carbone par volume dans le gaz de synthèse étant $(H_2 - CO_2)/(CO + CO_2) = 2{,}0$ à $2{,}05$.

**4.** Procédé selon la revendication 1, **caractérisé en ce que**, avant la désulfurisation du mélange de gaz, le procédé comprend en outre la pressurisation du mélange de gaz à une pression de 2,2 MPa à 2,8 MPa.

**5.** Procédé selon la revendication 1, **caractérisé par** la mise en réaction du gaz de synthèse dans les conditions de 215 °C à 280 °C et de 5,5 MPa à 6,5 MPa pour produire du méthanol.

**6.** Procédé selon la revendication 5, **caractérisé par** la mise en réaction du gaz de synthèse pour produire du méthanol et en même temps l'ajout d'eau pour compléter la perte d'humidité dans la réaction, et le rapport en poids entre la quantité d'eau ajoutée et le rendement en méthanol est de 0,90 à 0,96:1.

**7.** Procédé selon l'une quelconque des revendications 1 à 6, **caractérisé en ce qu'**il comprend en outre la rectification du méthanol pour obtenir du méthanol raffiné.

**8.** Dispositif de production de méthanol, **caractérisé en ce qu'**il comprend :

• un dispositif pour la purification de gaz de convertisseur relié à un orifice d'échappement du gaz de convertisseur ; un dispositif de mélange relié à la conduite de gaz de cokerie et à un orifice d'échappement du dispositif pour la purification du gaz de convertisseur ;
• un dispositif de synthèse relié à l'orifice d'échappement du dispositif de mélange ; et
• un dispositif de régulation relié au dispositif de synthèse, à la conduite de gaz de cokerie et à l'orifice d'échappement du dispositif pour la purification du gaz de convertisseur,

dans lequel le dispositif de régulation ajuste le débit du gaz de convertisseur purifié et du gaz de cokerie en se basant sur la surveillance en temps réel du rapport d'atomes d'hydrogène aux atomes de carbone dans le gaz de synthèse afin de réguler le rapport de mélange du gaz de convertisseur purifié et du gaz de cokerie.

**9.** Dispositif selon la revendication 8, **caractérisé en ce que** le dispositif de synthèse comprend :

• un dispositif de désulfuration relié à l'orifice d'échappement du dispositif de mélange ;
• un dispositif de reformage relié à l'orifice d'échappement du dispositif de désulfuration ; et
• une tour de synthèse reliée à l'orifice d'échappement du dispositif de reformage.

**12** CONVERTER GAS → DEVICE FOR PURIFYING CONVERTER GAS — 1

**13** COKE OVEN GAS → MIXING DEVICE — 2 → COMPRESSOR OF MIXED GAS — 3 → DEVICE FOR DESULFURATING — 4 → DEVICE FOR CONVERTING — 5

**11** OXYGEN

**14** REFINING METHANOL ← ATMOSPHERIC TOWER — 10 ← PRESSURIZED TOWER — 9 ← PRE-RECTIFICATION TOWER — 8 ← SYNTHESIS TOWER — 7 ← COMPRESSOR OF SYNTHESIS GAS — 6

**15** RELAXATION RELEASE GAS

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1373113

- GB 3382004 A

**Non-patent literature cited in the description**

- **LI KEBING et al.** *Chem Ind. Eng, Progress,* 2010, vol. 29, 325-327